# EUROPÄISCHE PATENTSCHRIFT

(11) **EP 3 810 080 B1**
(45) Veröffentlichungstag und Bekanntmachung des Hinweises auf die Patenterteilung: **20.11.2024**
(21) Anmeldenummer: 19731915.5
(22) Anmeldetag: 13.06.2019
(51) Int. Cl.: A61K 8/44, A61K 8/60, A61K 8/02, A61Q 19/10

(54) **MILDE KOSMETISCHE REINIGUNGSZUBEREITUNG**
MILD COSMETIC CLEANSING PREPARATION
PRÉPARATION NETTOYANTE COSMÉTIQUE DOUCE

(30) Priorität: 22.06.2018 DE 102018210175
(43) Veröffentlichungstag der Anmeldung: 28.04.2021
(73) Patentinhaber: Beiersdorf AG, 22529 Hamburg (DE)
(72) Erfinder: MEIRING, Uta, 21077 Hamburg (DE); NILSSON, Jan, 22177 Hamburg (DE)
(74) Vertreter: Beiersdorf AG
(86) Internationale Anmeldenummer: PCT/EP2019/065470
(87) Internationale Veröffentlichungsnummer: WO 2019/243151

(56) Entgegenhaltungen:
- EP-A1- 2 786 742
- WO-A2-2013/098066
- CN-A- 106 511 144
- DE-A1- 102013 206 314
- DE-A1- 3 724 460
- KR-A- 20160 059 263
- DATABASE GNPD [online] MINTEL; 28 August 2012 (2012-08-28), ANONYMOUS: "Shampoo", XP055878221, retrieved from https://www.gnpd.com/sinatra/recordpage/1864980/ Database accession no. 1864980
- DATABASE GNPD [online] MINTEL; 13 November 2014 (2014-11-13), ANONYMOUS: "Cleansing Cream for the Face", XP055878233, retrieved from https://www.gnpd.com/sinatra/recordpage/2772023/ Database accession no. 2772023
- DATABASE GNPD [online] MINTEL; 18 August 2015 (2015-08-18), ANONYMOUS: "Shampoo", XP055878236, retrieved from https://www.gnpd.com/sinatra/recordpage/3366011/ Database accession no. 3366011
- DATABASE GNPD [online] MINTEL; 9 January 2017 (2017-01-09), ANONYMOUS: "Foaming Cleanser", XP055878413, retrieved from https://www.gnpd.com/sinatra/recordpage/4535939/ Database accession no. 4535939
- DATABASE GNPD [online] MINTEL; 12 April 2017 (2017-04-12), ANONYMOUS: "Wash", XP055878414, retrieved from https://www.gnpd.com/sinatra/recordpage/4750187/ Database accession no. 4750187
- ANONYMOUS: "Polysorbat 20", WIKIPEDIA, 2 March 2022 (2022-03-02), pages 1 - 3, XP093097722, Retrieved from the Internet <URL:https://de.wikipedia.org/wiki/Polysorbat_20> [retrieved on 20231103]
- ANONYMOUS: "Polysorbat 80", WIKIPEDIA, 28 February 2022 (2022-02-28), pages 1 - 4, XP093097723, Retrieved from the Internet <URL:https://de.wikipedia.org/w/index.php?title=Polysorbat_80&oldid=220671491> [retrieved on 20231103]
- ALTMEYER PETER: "Glycereth-25- pca isostearates (inci)", ALTMEYERS ENCYCLOPEDIA, 29 October 2020 (2020-10-29), pages 1 - 4, XP093097724, Retrieved from the Internet <URL:https://www.altmeyers.org/en/cosmetology/glycereth-25-pca-isostearates-inci-144775> [retrieved on 20231103]
- ANONYMOUS: "BESCHLUSS DER KOMMISSION vom 9. Dezember 2014 zur Festlegung der Umweltkriterien für die Vergabe des EU-Umweltzeichens für "Rinse-off"-Kosmetikprodukte", AMTSBLATT DER EUROPÄISCHEN UNION, 11 December 2014 (2014-12-11), pages L 354/47 - L 354/61, XP093097726, Retrieved from the Internet <URL:https://eur-lex.europa.eu/legal-content/DE/TXT/PDF/?uri=CELEX:32014D0893> [retrieved on 20231103]
- ANONYMOUS: "Product Safety Assessment NEOLONE(TM) PE Preservative", PRODUCT SAFETY ASSESSMENT, 6 December 2011 (2011-12-06), pages 1 - 7, XP093097728, Retrieved from the Internet <URL:https://www.soapmakingstudio.com/soapmaking-supplies/sds/preservatives/neolone-pe.pdf>
- DATABASE GNPD [online] MINTEL; 22 September 2017 (2017-09-22), ANONYMOUS: "Shampoo", XP055878228, retrieved from https://www.gnpd.com/sinatra/recordpage/5116041/ Database accession no. 5116041
- DATABASE GNPD [online] MINTEL; November 2016 (2016-11-01), "Mild Bath & Shampoo", XP002795300, Database accession no. 4369333
- "EcoSense Surfactants - Dow", April 2013 (2013-04-01), XP002795311, Retrieved from the Internet <URL:http://msdssearch.dow.com/PublishedLiteratureDOWCOM/dh_08d4/0901b803808d4a93.pdf> [retrieved on 20191029]

## Beschreibung

Die vorliegende Erfindung betrifft eine kosmetische Reinigungszubereitung enthaltend
a) ein oder mehrere Glycolipide (INCI: Glycolipids)
b) mindestens zwei oberflächenaktiven Verbindungen gewählt aus der Gruppe Cocamidopropylbetain, Natriumcocoamphoacetat, Laurylglucosid, Decylglucosid, Coco-Glucoside mit Ausnahme der Kombination aus Natriumcocoamphoacetat und Laurylglucoside und mit der Maßgabe, dass bei der Kombination aus Cocamidopropylbetain und Natriumcocoamphoacetat die Zubereitung frei ist von Laurylsulfosuccinaten, sowie ein Tuch, welches mit dieser Zubereitung getränkt ist.

Die Produktion von kosmetischen Reinigungsmitteln zeigt seit Jahren eine steigende Tendenz. Dies ist vor allem auf das zunehmende Gesundheitsbewusstsein und Hygienebedürfnis der Verbraucher zurückzuführen.

Reinigung des menschlichen Körpers bedeutet das Entfernen von (Umwelt-) Schmutz und bewirkt damit eine Erhöhung des psychischen und physischen Wohlbefindens. Die Reinigung der Oberfläche von Haut und Haaren ist ein sehr komplexer, von vielen Parametern abhängiger Vorgang. Zum einen sollen von außen kommende Substanzen wie beispielsweise Kohlenwasserstoffe oder anorganische Pigmente aus unterschiedlichsten Umfeldern sowie Rückstände von Kosmetika oder auch unerwünschte Mikroorganismen möglichst vollständig entfernt werden. Zum anderen sind körpereigene Ausscheidungen wie Schweiß, Sebum, Haut- und Haarschuppen ohne tiefgreifende Eingriffe in das physiologische Gleichgewicht abzuwaschen.

Kosmetische Reinigungspräparate sind sogenannte "rinse off" Präparate, welche nach der Anwendung von der Haut abgespült werden. Sie werden in aller Regel in Form eines Schaums mit Wasser auf die zu reinigenden Körperpartien aufgetragen. Basis aller kosmetischen oder dermatologischen Reinigungspräparate sind waschaktive Tenside. Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfernung und -lösung, ein leichtes Abspülen und - je nach Wunsch - auch für Schaumregulierung. Damit ist die Grundlage für die Schmutzentfernung lipidhaltiger Verschmutzungen gegeben.

Üblicherweise müssen kosmetische Reinigungszubereitungen einen relativ hohen Anteil an gut schäumenden Tensiden (z.B. Seifen, Natriumlaurylethersulfat) enthalten, die benötigt werden, um Luft in die Zubereitung einzutragen und diese aufzuschäumen. Ferner sollten Reinigungszubereitungen, wenn sie nicht fest bereits vorliegen, zu einem Gel verdickt werden, um bei der Anwendung leichter handhabbar zu sein.

Nach dem Stande der Technik kennt der Fachmann verschiedene Lösungswege, um kosmetische Reinigungsgele herzustellen.

Zum einen kann er Laurylethersulfat-haltige Zubereitungen einsetzen, die durch Zusatz von Salz eine Gel-artige Konsistenz erhalten. Dieser Lösungsweg hat jedoch den Nachteil, dass nicht besonders hautverträgliche Tenside (Laurylethersulfate) eingesetzt werden. Dieser Lösungsweg ist damit keine Option für Menschen mit empfindlicher Haut, beispielsweise Babies.

Zum anderen kann der Fachmann auf Systeme mit Polyethylenglycol-Verbindungen (PEG-Verbindungen) zurückgreifen, beispielsweise ethoxylierte Fettalkohole (z.B. PEG-120 Methylglucose Dioleate), PEG-200 Hydrogenated Glyceryl Palmitate. Dieser Lösungsweg hat den Nachteil, dass die beim Verbraucher unbeliebten PEG-Verbindungen eingesetzt werden, von denen einige Forscher vermuten, dass sie aus toxikologischer Sicht nicht ganz unbedenklich sein könnten. Auch für diesen Lösungsweg gilt, dass er für Menschen mit empfindlicher Haut, beispielsweise Babies, keine wirklich befriedigende Option darstellt. Ferner kann der Fachmann auf Biopolymere wie Xanthan Gum, Zellulosen oder Stärken zurückgreifen. Beim Einsatz dieser Verbindungen tritt dann das Problem auf, dass die wässrigen Zubereitungen mit hohen Konzentrationen an Konservierungsmitteln versetzt werden müssen, um vor mikrobieller Zersetzung geschützt zu werden. Da der Einsatz von Konservierungsmitteln beim Verbraucher ebenfalls aus diversen Gründen unerwünscht und mit Skepsis betrachtet wird, stellt dieser Lösungsweg bisher keine befriedigende Option für Menschen mit empfindlicher Haut, beispielsweise Babies, dar.

Nicht zuletzt kann der Fachmann auch auf synthetisch hergestellte Polymere zurückgreifen, deren Einsatz aufgrund ihrer langsamen biologischen Abbaubarkeit aus Umweltschutzgründen vermieden werden sollte.

Es war daher die Aufgabe der vorliegenden Erfindung, eine besonders hautfreundliche, milde und gut schäumende verdickte Reinigungszubereitung herzustellen, die möglichst auf umstrittene Inhaltsstoffe wie PEG-Verbindungen und Konservierungsmittel verzichten kann. Die Zubereitung sollte relativ gut biologisch abbaubar sein und idealerweise einen möglichst großen Anteil an Inhaltsstoffen enthalten, die auf nachwachsenden Rohstoffen basieren. Überraschend gelöst wird die Aufgabe durch eine kosmetische Reinigungszubereitung gemäß Anspruch 1.

Zwar kennt der Fachmann die Offenbarungen WO 2013/098066, DE 102013206314, EP2786742, den Datenbank-Eintrag in der GNPD Datenbank "Mintel" "Mild Bath & Shampoo" (Eintragungsnummer 4369333) und die Internet-Publikation zu "EcoSense Surfactants-Dow" vom April 2013 der Firma Dow, doch konnten diese Schriften nicht den Weg zur vorliegenden Erfindung weisen.

Dabei war es für den Fachmann insbesondere überraschend, dass die viskositätssteigernde Wirkung allein auf der Kombination der eingesetzten Tenside basiert und ein Zusatz weiterer Verdickungsmittel nicht mehr notwendig ist.

Ferner war es für den Fachmann nicht naheliegend, dass die Zubereitungen in der Lage sind, ausreichend Micellen zu bilden.

Diese Kombination an Inhaltsstoffen zeigt eine relativ hohe Viskosität, während die Einzelkomponenten die Zubereitung nicht verdicken können.

Sie ist insbesondere vorteilhaft, um Zubereitungen mit einer Viskosität von 1200 bis 1600 mPas (Rheomat proRheo 123, Spindel 1, 25°C, 62,5rpm) herzustellen.

Es ist erfindungsgemäß bevorzugt, wenn die erfindungsgemäße Zubereitung einen pH-Wert von kleiner oder gleich 5 aufweist. In einem solchen Fall kann die Einsatzmenge an Konservierungsmitteln stark reduziert werden.

Überraschend war es für den Fachmann dabei, dass die erfindungsgemäßen Zubereitungen auch noch in diesem relativ niedrigen pH-Bereich noch eine ausreichende Viskosität aufweisen und transparent sind, während viele Zubereitungen des Standes der Technik (z.B. mit Polyacrylaten verdickte Zubereitungen) in diesem pH-Bereich nicht mehr verdicken und trüb sind.

Glycolipide stellen natürliche Verbindungen dar, die aus Zucker und Fettsäuren gebildet werden. Die erfindungsgemäßen Glycolipide können im Fermentationsprozess aus Zucker gewonnen werden. Sie sind beispielsweise bei der Firma Evonik unter dem Handelsnamen "Rheance^{®} One" erhältlich. Da dieser Rohstoff üblicherweise mit ca. 0,6 Gew.-% Natriumbenzoat konserviert ist, ist dieser Stoff üblicherweise auch in den erfindungsgemäßen Zubereitungen nachweisbar.

Erfindungsgemäß ist die kosmetische Reinigungszubereitung, dadurch gekennzeichnet, dass die Zubereitung Glycolipide (INCI: Glycolipids) mit einem Aktivgehalt von mindestens 2 Gewichts-%, bezogen auf das Gesamtgewicht der Zubereitung enthält. Erfindungsgemäß bevorzugt ist dabei der Aktivgehalt von 4 bis 6 Gewichts-%, bezogen auf das Gesamtgewicht der Zubereitung.

Ferner ist es erfindungsgemäß von Vorteil, wenn die erfindungsgemäße Zubereitung Cocamidopropylbetain (INCI: Cocamidopropyl betaine) mit einem Aktivgehalt von mindestens 2

Gewichts-%, bezogen auf das Gesamtgewicht der Zubereitung enthält. Erfindungsgemäß bevorzugt ist dabei der Aktivgehalt von 5 bis 7 Gewichts-%, bezogen auf das Gesamtgewicht der Zubereitung.

Es ist vorteilhaft im Sinne der vorliegenden Erfindung, wenn die Zubereitung Natriumcocoamphoacetat (INCI: Sodium Cocoamphoacetate) mit einem Aktivgehalt von mindestens 2 Gewichts-%, bezogen auf das Gesamtgewicht der Zubereitung enthält. Erfindungsgemäß bevorzugt ist dabei der Aktivgehalt von 5 bis 7 Gewichts-%, bezogen auf das Gesamtgewicht der Zubereitung.

Für Decylglucosid ist es erfindungsgemäß von Vorteil, wenn die Zubereitung Decylglucosid (INCI: Decyl Glucoside) mit einem Aktivgehalt von mindestens 2 Gewichts-%, bezogen auf das Gesamtgewicht der Zubereitung enthält. Erfindungsgemäß bevorzugt ist dabei der Aktivgehalt von 5 bis 7 Gewichts-%, bezogen auf das Gesamtgewicht der Zubereitung.

Erfindungsgemäße Zubereitungen sind dadurch gekennzeichnet, dass die Zubereitung Phenoxyethanol, Ethylhexylglycerin, Butylenglycol, 2-Methylpropan-1,3-diol, 1,2-Pentandiol, 1,2-Hexandiol, 1,2-Octandiol und/oder 1,2-Decandiol enthält. Dabei wird, erfindungsgemäß bevorzugt Zubereitung Phenoxyethanol, Ethylhexylglycerin,
Butylenglycol, 2-Methylpropan-1,3-diol, 1,2-Pentandiol, 1,2-Octandiol und/oder 1,2-Decandiol.

Ferner ist es erfindungsgemäß von Vorteil, wenn die Zubereitung Xylitylsesquicaprylate, Hydroxyacetophenone, Glyceryl Caprylate, Natrium Chlorid, Natrium Benzoate, Natrium Salicylate, Dehydroacetic Acid, Anisic Acid, Levulinic Acid, Hydroxypropyl Guar Hydroxypropyl Trimonium Chlorid, Polyquaternium-7, Polyquaternium-10 und/oder Lecithin enthält.

Vorteilhaft im Sinne der vorliegenden Erfindung ist es auch, wenn die erfindungsgemäße Zubereitung einen oder mehrere Wirkstoffe aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Polydocanol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält. Dabei ist insbesondere der Einsatz von Panthenol und/oder Tocopherylacetat bevorzugt.

Die erfindungsgemäße Zubereitung kann darüber hinaus erfindungsgemäß vorteilhaft Glycerin enthalten. Dieses wird üblicherweise in einer Konzentration von 0,01 bis 10 Gewichts-%, bezogen auf das Gesamtgewicht der Zubereitung eingesetzt.

Ferner kann die erfindungsgemäße Zubereitung darüber hinaus erfindungsgemäß vorteilhaft Ethanol enthalten. Dieses wird üblicherweise in einer Konzentration von 0,01 bis 5,0 Gewichts-%, bezogen auf das Gesamtgewicht der Zubereitung eingesetzt.

Darüber hinaus kann die erfindungsgemäße Zubereitung mit für die Kosmetik zugelassenen Farbstoffen wie CI 74160, CI 61570, CI 42090, CI 42051, CI 47005, CI 13015, CI 10316, CI 19140, CI 15985, CI 17200, CI 14720, CI 28440, Cl 14720, CI 19140, CI 28440, CI 61565, CI 42053, CI 16035, CI 51319, Cl 77491, Cl 77891 einfärbt sein.

Die erfindungsgemäßen Zubereitungen sind, insbesondere wenn sie Micellen bilden, transparent. Dabei gilt eine Zubereitung erfindungsgemäß und anspruchsgemäß als transparent, wenn es möglich ist, bei Tageslicht durch eine mit der erfindungsgemäßen Zubereitung gefüllten Einmal-Küvette (Firma Brand, 2,5ml, Wellenlängenbereich: 220nm-900nm) mit dem bloßen Auge zu schauen. Schriftzeichen (Schrifttyp Arial Schriftgröße 10), die sich unmittelbar hinter der Einmal-Küvette befinden, sollten erkennbar und lesbar sein.

Ferner kann es erfindungsgemäß von Vorteil sein, wenn die erfindungsgemäße Zubereitung einen oder mehrere Parfümstoffe enthält. Diese können beispielsweise gewählt werden aus der Liste der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin. Erfindungsgemäß ist auch ein kosmetisches Reinigungstuch getränkt mit einer erfindungsgemäßen Reinigungszubereitung.

In einem solchen Fall ist es erfindungsgemäß bevorzugt, wenn das Tuchmaterial Viskosefasern, PET, Polylacticacid (PLA), Cellulose, Mischung aus Celluloseacetat mit Viskosefasern (Tencel) oder Baumwollfasern enthält.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| Tränkungsmedium für wipes (Viskosität vorzugsweise <500mpas) | | |
|---|---|---|
| Tränkungsgrad | Grammage g/m2 | Tuchmaterial |
| 3,1 | 43 | 100% Viskose |
| 2,8 | 50 | Tencel |
| 2,5 | 43 | PET |

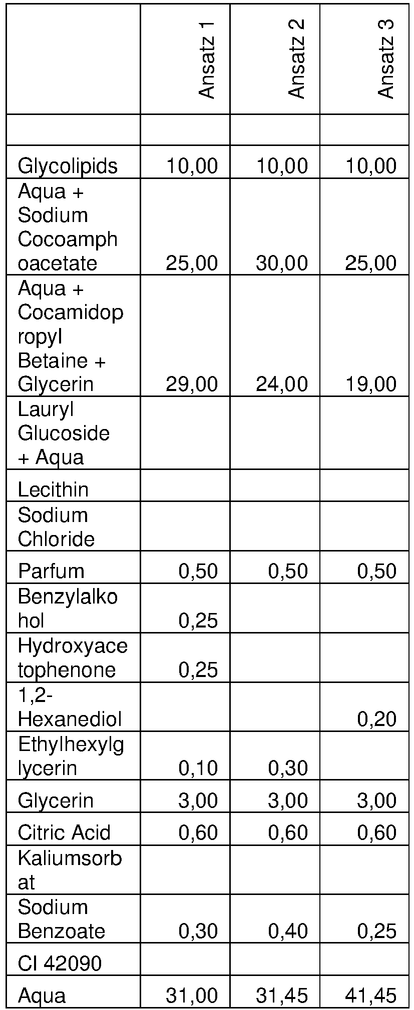

Die folgenden Ansätze 11-17liegen außerhalb der Erfindung.

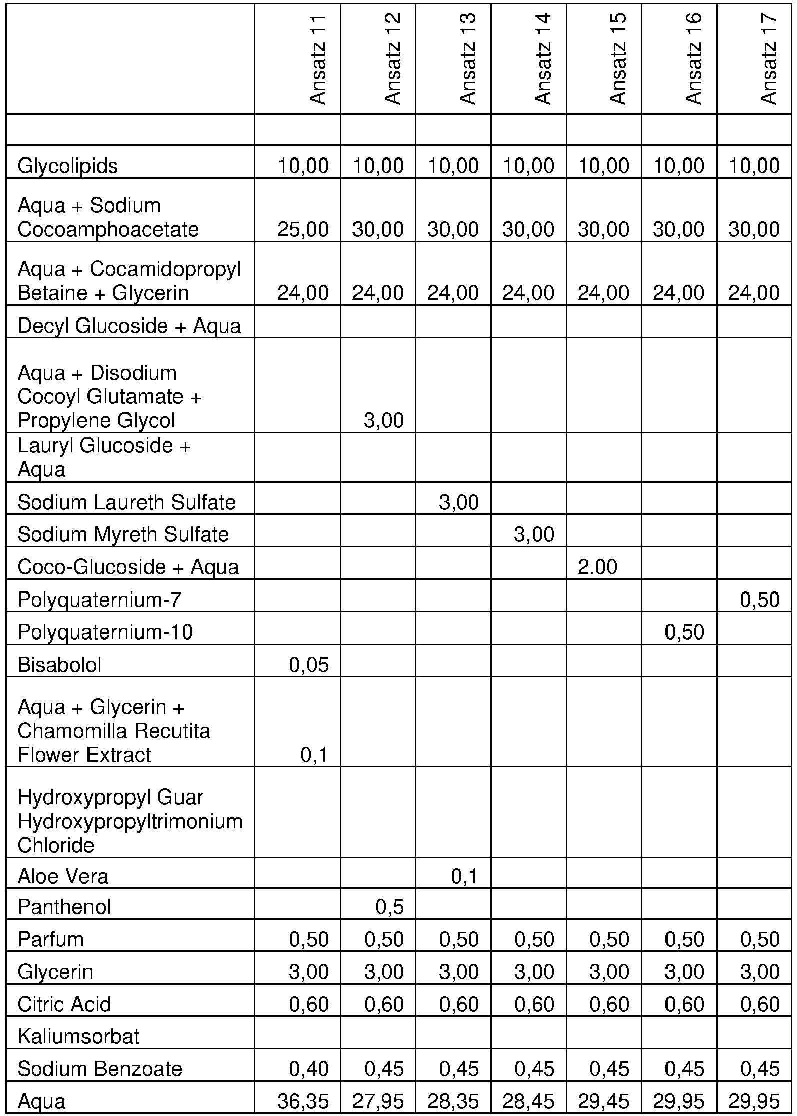

## Patentansprüche

1. Kosmetische Reinigungszubereitung enthaltend
a) ein oder mehrere Glycolipide mit der INCI Bezeichnung Glycolipids,
b) mindestens zwei oberflächenaktiven Verbindungen gewählt aus der Gruppe Cocamidopropylbetain, Natriumcocoamphoacetat, Laurylglucosid, Decylglucosid, Coco-Glucoside mit Ausnahme der Kombination aus Natriumcocoamphoacetat und Laurylglucoside und mit der Maßgabe, dass bei der Kombination aus Cocamidopropylbetain und Natriumcocoamphoacetat die Zubereitung frei ist von Laurylsulfosuccinaten, wobei die Zubereitung Phenoxyethanol, Ethylhexylglycerin, Butylenglycol, 2-Methylpropan-1,3-diol, 1,2-Pentandiol, 1,2-Hexandiol, 1,2-Octandiol und/oder 1,2-Decandiol enthält und die Zubereitung frei ist von Polyethylenglycol-Fettalkoholethern oder -estern, Acylisethionaten, Sulfosuccinaten, Propyl- und Butylparaben, 3-lod-2-propinylbutylcarbamat, DMDM Hydantoin, Methylisothiazolinon, Chlormethylisothiazolinon und Hydroxyisohexyl 3-Cyclohexene Carboxalhdehyde, wobei die Zubereitung Glycolipide mit der INCI-Bezeichnung Gylcolipids mit einem Aktivgehalt von mindestens 2 Gewichts-%, bezogen auf das Gesamtgewicht der Zubereitung enthält, **dadurch gekennzeichnet, dass** die Zubereitung eine Kombination aus
a) ein oder mehreren Glycolipiden mit der INCI Bezeichnung Glycolipids,
b) Cocamidopropylbetain mit der INCI-Bezeichnung Cocamidopropyl Betaine und
c) Natriumcocoamphoacetat mit der INCI-Bezeichnnung Sodium Cocoamphoacetate enthält.

2. Kosmetische Reinigungszubereitung nach Anspruch 1, **dadurch gekennzeichnet, dass** die Zubereitung einen pH-Wert von kleiner oder gleich 5 aufweist.

3. Kosmetische Reinigungszubereitung nach einem der vorhergehenden Ansprüche, **dadurch gekennzeichnet, dass** die Zubereitung Cocamidopropylbetain mit der INCI-Bezeichnung Cocamidopropyl Betaine mit einem Aktivgehalt von mindestens 2 Gewichts-%, bezogen auf das Gesamtgewicht der Zubereitung enthält.

4. Kosmetische Reinigungszubereitung nach einem der vorhergehenden Ansprüche, **dadurch gekennzeichnet, dass** die Zubereitung Natriumcocoamphoacetat mit der INCI-Bezeichnung Sodium Cocoamphoacetate mit einem Aktivgehalt von mindestens 2 Gewichts-%, bezogen auf das Gesamtgewicht der Zubereitung enthält.

5. Kosmetische Reinigungszubereitung nach einem der vorhergehenden Ansprüche, **dadurch gekennzeichnet, dass** die Zubereitung Decylglucosid mit der INCI-Bezeichnung Decyl Glucoside mit einem Aktivgehalt von mindestens 2 Gewichts-%, bezogen auf das Gesamtgewicht der Zubereitung enthält.

6. Kosmetische Reinigungszubereitung nach einem der vorhergehenden Ansprüche, **dadurch gekennzeichnet, dass** die Zubereitung Xylitylsesquicaprylate, Hydroxyacetophenone, Glyceryl Caprylate, Natrium Chlorid, Natrium Benzoate, Natrium Salicylate, Dehydroacetic Acid, Anisic Acid, Levulinic Acid, Hydroxypropyl Guar Hydroxypropyl Trimonium Chlorid, Polyquaternium-7, Polyquaternium-10 und/oder Lecithin enthält

7. Kosmetische Reinigungszubereitung nach einem der vorhergehenden Ansprüche, **dadurch gekennzeichnet, dass** die Zubereitung einen oder mehrere Wirkstoffe aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Polydocanol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

8. Kosmetische Reinigungstuch getränkt mit einer Reinigungszubereitung nach einem der vorhergehenden Ansprüche.

9. Reinigungstuch nach Anspruch 8, **dadurch gekennzeichnet, dass** das Tuchmaterial Viskosefasern enthält.

## Claims

1. Cosmetic cleansing preparation comprising
a) one or more glycolipids with the INCI name Glycolipids,
b) at least two surface-active compounds selected from the group of cocamidopropyl betaine, sodium cocoamphoacetate, lauryl glucoside, decyl glucoside, coco-glucoside with the exception of the combination of sodium cocoamphoacetate and lauryl glucoside and with the proviso that, in the case of the combination of cocamidopropyl betaine and sodium cocoamphoacetate, the preparation is free of lauryl sulfosuccinates, the preparation comprising phenoxyethanol, ethylhexylglycerin, butylene glycol, 2-methylpropane-1,3-diol, petane-1,2-diol, hexane-1,2-diol, octane-1,2-diol and/or decane-1,2-diol and the preparation being free of polyethylene glycol fatty alcohol ethers or esters, acyl isethionates, sulfosuccinates, propyl and butyl paraben, 3-iodo-2-propynyl butylcarbamate, DMDM hydantoin, methylisothiazolinone, chloromethylisothiazolinone and Hydroxyisohexyl 3-Cyclohexene Carboxaldehyde, the preparation comprising glycolipids with the INCI name Glycolipids with an active content of at least 2% by weight, based on the total weight of the preparation, **characterized in that** the preparation comprises a combination of
a) one or more glycolipids with the INCI name Glycolipids,
b) cocamidopropyl betaine with the INCI name Cocamidopropyl Betaine and
c) sodium cocoamphoacetate with the INCI name Sodium Cocoamphoacetate.

2. Cosmetic cleansing preparation according to Claim 1, **characterized in that** the preparation has a pH of less than or equal to 5.

3. Cosmetic cleansing preparation according to either of the preceding claims, **characterized in that** the preparation comprises cocamidopropyl betaine with the INCI name Cocamidopropyl Betaine with an active content of at least 2% by weight, based on the total weight of the preparation.

4. Cosmetic cleansing preparation according to any of the preceding claims, **characterized in that** the preparation comprises sodium cocoamphoacetate with the INCI name Sodium Cocoamphoacetate with an active content of at least 2% by weight, based on the total weight of the preparation.

5. Cosmetic cleansing preparation according to any of the preceding claims, **characterized in that** the preparation comprises decyl glucoside with the INCI name Decyl Glucoside with an active content of at least 2% by weight, based on the total weight of the preparation.

6. Cosmetic cleansing preparation according to any of the preceding claims, **characterized in that** the preparation comprises Xylityl Sesquicaprylate, Hydroxyacetophenone, Glyceryl Caprylate, Sodium Chloride, Sodium Benzoate, Sodium Salicylate, Dehydroacetic Acid, Anisic Acid, Levulinic Acid, Hydroxypropyl Guar Hydroxypropyltrimonium Chloride, Polyquaternium-7, Polyquaternium-10 and/or Lecithin.

7. Cosmetic cleansing preparation according to any of the preceding claims, **characterized in that** the preparation comprises one or more active ingredients from the following group of compounds: alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, panthenol, polydocanol, magnolol, honokiol, tocopheryl acetate, dihydroxyacetone; 8-hexadecene-1,16-dicarboxylic acid, glycerylglucose, (2-hydroxyethyl)urea, vitamin E and derivatives thereof, hyaluronic acid and/or salts thereof, and/or licochalcone A.

8. Cosmetic cleansing wipe impregnated with a cleansing preparation according to any of the preceding claims.

9. Cleansing wipe according to Claim 8, **characterized in that** the wipe material comprises viscose fibres.

## Revendications

1. Préparation nettoyante cosmétique contenant
a) un ou plusieurs glycolipides ayant la désignation INCI Glycolipids,
b) au moins deux composés tensioactifs choisis dans le groupe cocoamidopropylbétaïne, cocoamphoacétate de sodium, laurylglucoside, décylglucoside, coco-glucosides à l'exception de la combinaison de cocoamphoacétate de sodium et de laurylglucosides, et à la condition que, lors de la combinaison de cocoamidopropylbétaïne et de cocoamphoacétate de sodium, la préparation soit exempte de laurylsulfosuccinates, la préparation contenant du phénoxyéthanol, de l'éthylhexylglycérol, du butylèneglycol, du 2-méthylpropane-1,3-diol, du 1,2-pentanediol, du 1,2-hexanediol, du 1,2-octanediol et/ou du 1,2-décanediol et la préparation étant exempte d'alcools gras ou d'esters d'alcools gras polyéthoxylés, d'acylisothionates, de sulfosuccinates, de propyl- et de butylparaben, de 3-iodo-2-propynylbutylcarbamate, de DMDM hydantoïne, de méthylisothiazolinone, de chlorométhylisothiazolinone et d'hydroxyisohexyle 3-cyclohexènes carboxaldéhydes, la préparation contenant des glycolipides ayant la désignation INCI Glycolipids, avec une teneur en actifs d'au moins 2 % en poids par rapport au poids total de la préparation, **caractérisée en ce que** la préparation contient une combinaison de
a) un ou plusieurs glycolipides ayant la désignation INCI Glycolipids,
b) cocamidopropylbétaïne ayant la désignation INCI Cocamidopropyl Betaine et
c) cocoamphoacétate de sodium ayant la désignation INCI Sodium Cocoamphoacetate.

2. Préparation nettoyante cosmétique selon la revendication 1, **caractérisée en ce que** la préparation a un pH inférieur ou égal à 5.

3. Préparation nettoyante cosmétique selon l'une des revendications précédentes, **caractérisée en ce que** la préparation contient de la cocamidopropylbétaïne ayant la désignation INCI Cocamidopropyl Betaine avec une teneur en actifs d'au moins 2 % en poids par rapport au poids total de la préparation.

4. Préparation nettoyante cosmétique selon l'une des revendications précédentes, **caractérisée en ce que** la préparation contient du cocoamphoacétate de sodium ayant la désignation INCI Sodium Cocoamphoacetate avec une teneur en actifs d'au moins 2 % en poids par rapport au poids total de la préparation.

5. Préparation nettoyante cosmétique selon l'une des revendications précédentes, **caractérisée en ce que** la préparation contient du décylglucoside ayant la désignation INCI Decyl Glucoside avec une teneur en actifs d'au moins 2 % en poids par rapport au poids total de la préparation.

6. Préparation nettoyante cosmétique selon l'une des revendications précédentes, **caractérisée en ce que** la préparation contient du sesquicaprylate de xylityle, de l'hydroxyacétophénone, du caprylate de glycéryle, du chlorure de sodium, du benzoate de sodium, du salicylate de sodium, de l'acide déshydroacétique, de l'acide anisique, de l'acide lévulinique, du chlorure d'hydroxypropyl guar hydroxypropyl trimonium, du polyquaternium-7, du polyquaternium-10 et/ou de la lécithine.

7. Préparation nettoyante cosmétique selon l'une des revendications précédentes, **caractérisée en ce que** la préparation contient une ou plusieurs matières actives du groupe des composés acide alpha-lipoïque, acide folique, phytoène, D-biotine, coenzyme Q10, alpha-glucosylrutine, carnitine, carnosine, isoflavonoïde naturel et/ou synthétique, flavonoïde, créatine, créatinine, taurine, β-alanine, panthénol, polydocanol, magnolol, honokiol, acétate de tocophéryle, dihydroxyacétone ; acide 8-hexadécène-1,16-dicarboxylique, glycérylglycose, (2-hydroxyéthyl)urée, vitamin E ou ses dérivés, acide hyaluronique et/ou ses sels et/ou licochalcone A.

8. Chiffon nettoyant cosmétique imprégné d'une préparation nettoyante selon l'une des revendications précédentes.

9. Chiffon nettoyant selon la revendication 8, **caractérisé en ce que** le matériau du chiffon contient des fibres de viscose.
